# EUROPEAN PATENT SPECIFICATION

(11) **EP 1 051 221 B1**
(45) Date of publication and mention of the grant of the patent: **14.01.2004**
(21) Application number: 99900593.7
(22) Date of filing: 27.01.1999
(51) Int. Cl.: A61N 1/36, A61N 1/08

(54) **MUSCLE ELECTROSTIMULATOR CONFORMED IN SUCH A WAY AS TO AVOID SENSATIONS OF PAIN AND REDDENING OF THE SKIN**
ELEKTRISCHER MUSKELSTIMULATOR MIT EINER SCHALTUNGSANORDNUNG ZUR VERHINDERUNG VON SCHMERZEN UND RÖTUNGEN DER HAUT
ELECTROSTIMULATEUR MUSCULAIRE EMPECHANT LES SENSATIONS DE DOULEUR ET LES ROUGISSURES DE LA PEAU

(30) Priority: 29.01.1998 IT PS980002 U
(43) Date of publication of application: 15.11.2000
(60) Divisional application: 03022037.0
(73) Proprietor: Baldi Holding S.A., 1118 Luxembourg (LU)
(72) Inventor: AGABITI, Gabriele, I-61100 Pesaro (IT); CORSI, Dario, I-61100 Pesaro (IT); PONSELE', Michele, I-61100 Pesaro (IT)
(74) Representative: Lanzoni, Luciano
(86) International application number: PCT/IB1999/000147
(87) International publication number: WO 1999/038567

(56) References cited:
- US-A- 4 088 141
- US-A- 4 189 685
- US-A- 4 580 570
- US-A- 5 504 420

## Description

### Technical Field

The present invention relates to a muscle stimulator able to improve the physical training of athletes, suitable for use in physical therapy in general and in particular when it is impossible to perform rehabilitation exercises with various types of equipment, or for use in aesthetic treatments.

In humans, muscle contraction takes place thanks to nerve stimuli sent voluntarily from the brain (central nervous system), which pass through the spinal cord and then through the motor nerves which innervate the various skeletal striated muscles in the body.

These muscles can also be stimulated to contract by external stimuli, such as that of an electrical shock.

Muscle electrostimulation is the practice which allows to obtain contractions of the skeletal striated muscles that are similar in every way to voluntary muscle contractions. This non-invasive practice, which can substitute the nerve signal sent by the central nervous system, can be utilised to develop the muscle characteristics of athletes, or in rehabilitation treatment for patients suffering from muscle atrophy due to the protracted lack of use of a set of muscles.

Electrostimulators generate electrical pulses which are sent to electrodes applied to the skin of the body parts to be treated. The way in which the pulses are used differs according to the individual, the type of muscle to be stimulated, and the characteristics of the skin to which the electrodes are to be applied.

### Background Art

The muscle electrostimulators currently in use have the drawback of supplying excess voltage which, in the case of accidental detachment of an electrode, can cause sensations of pain. This is because the breaking of the circuit, and the resulting increase in impedance towards infinite values, causes a sudden rise in the voltage supplied to the electrode by the generator, which attempts to maintain the current constant.

Another drawback of the devices currently in use is that of causing, in certain cases, the reddening of, or bums to, the skin near the electrodes.

Document US-A-4 088 141 discloses an electro stimulator comprising a constant current impulse generator connected to electrodes which, in turn, have to be applied onto the skin. Monitoring and input circuits, as well as regulating components are provided to control the generation of the constant current pulses. In particular a circuit is provided which monitors the impedance at the electrodes and blocks the output of current when said impedance exceeds a predetermined threshold value, in order to avoid sensation of pain or shocks to the user. Impedance at the electrodes is evaluated by measuring the voltage on the electrodes as well as the output current.

### Disclosure of Invention

The present invention has the purpose of allowing the effective electrostimulation of the various muscles of the body, avoiding sensations of the pain should an electrode become detached and avoiding the reddening or burning of the skin.

For this purpose, a monitoring circuit is used to control excess voltage on the electrodes by blocking the passage of current within an extremely brief time, during which the person is unable to feel any pain. By correctly repositioning the device, the emission of pulses is restored, with a voltage output which, in a ramped or in any case rising manner, increases from zero to the predetermined value, in such a way as not to cause any painful shocks.

By means of automatic temperature monitoring on the contact surface of the electrodes, the subject device also prevents excessive heating of the body part concerned and therefore the undesired reddening or burning of the skin. This allows the flow of current along the electrodes themselves to be limited or blocked in the case of an excessive rise in the temperature of the area whereto they are applied.

The invention shall now be described in detail, with reference to the accompanying plates and drawings, which illustrate a possible embodiment provided purely by way of example.

### Description of the Drawings

Figure 1, plate 1 shows a block diagram of the device.
Figure 2, plate 2 shows a possible version of the corresponding wiring diagram.

### Description of the Illustrative Embodiment

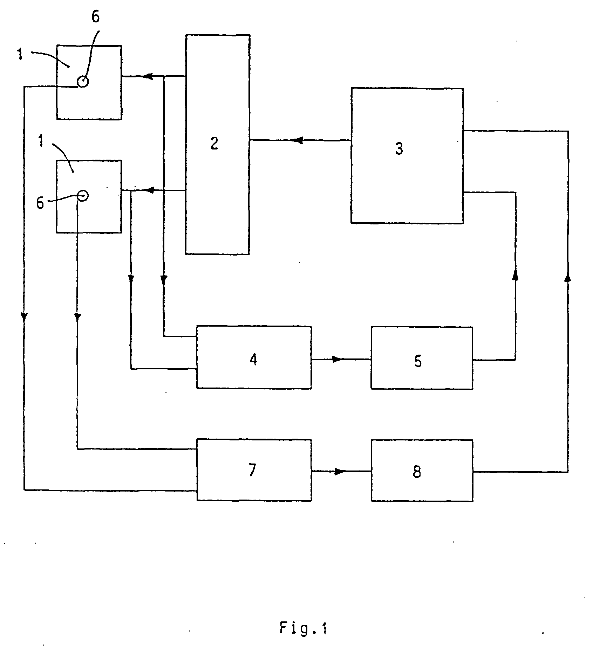

In Figure 1, the electrodes to be applied in variable number on the body parts involved are indicated with the reference number 1. These electrodes are connected to the pulse generator 2, which supplies electrical pulses whose characteristics are monitored by the central processing unit (CPU). The amplitude of the pulses sent to the electrodes is monitored with the peak detector 4, whose output is converted from analogue to digital form by means of the analogue to digital converter 5. The digital input of this converted, proportional to the amplitude of the pulses sent to the electrodes, is read by the CPU 3 which, based on this amplitude, acts on the pulse generator 2, in such a way as to block current output within 5 microseconds in the case of temporary excess voltage due to the accidental detachment of an electrode. When the electrode is repositioned correctly, the CPU restores pulse emission, ramping up the amplitude from zero to the maximum value set previously by means of potentiometers. In this way, there is no need to set voltage to zero manually by means of the regulating potentiometers, which thus remain positioned on the preselected optimal value, avoiding time wastage and the possibility of errors.

The number 6 indicates the temperature sensors applied inside the electrodes 1. The output signals of the sensors are amplified by amplifier 7 and converted to a digital signal by the analogue-to-digital converter 8. The CPU reads this output and, in case of an excessive temperature increase, it limits or blocks the pulses sent by the electrodes.

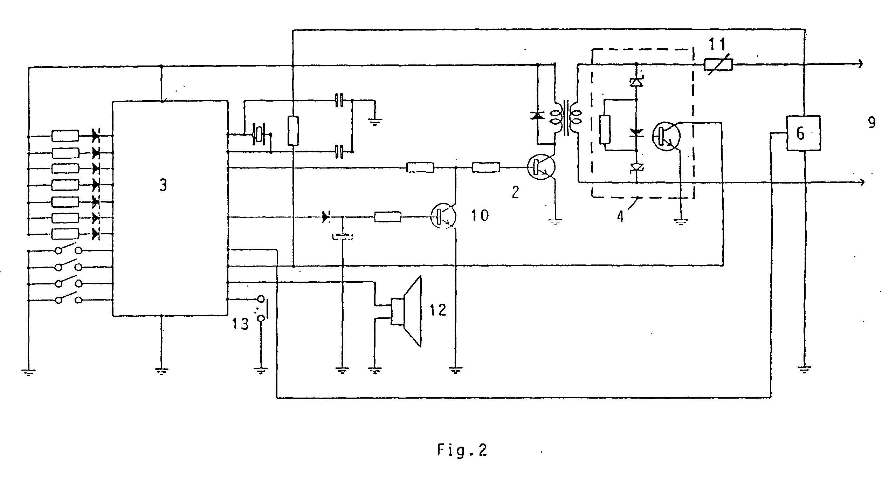

In Figure 2, the electrode terminals are indicated with the number 9. In this figure the same reference numbers as in Figure 1 are used to indicate the pulse generator 2, the CPU 3, the peak detector 4 and the temperature sensor 6. The analogue-to-digital converters and the amplifier are integrated into the CPU (but could obviously be separated). The ramp generator is indicated with the number 10 and the potentiometer for the regulation of the current sent to the electrode is indicated with the number 11. The acoustic alarm is indicated with the number 12 and the start/reset button is indicated with the number 13.

Obviously, the number of electrodes and the constructive details of the circuit could differ from those illustrated solely by way of example, without thereby departing from the scope of the present invention.

## Claims

1. Muscle electrostimulator conformed in such a way as to avoid sensations of pain and reddening of the skin, comprising an impulse generator (2), monitoring and input circuits, electrodes (1), regulating components (11) and a circuit (4,5,3) for monitoring excess voltage on the electrodes which, if the prescribed limit is exceeded, blocks the output of current within a time of such a duration that the person is unable to perceive any pain, means (10) for restoring the emission of the pulses after the electrode is correctly repositioned, **characterized in that** said means (10) for restoring the emission of pulses acting by ramping up the amplitude of the pulses from zero to a preselected value in such a way as to prevent painful shocks without the need to set voltage to zero manually by means of the regulating components (11) which thus remain positioned on the preselected optimal value.

2. Electrostimulator according to claim 1, **characterized in that**, it comprises a CPU (3), said means (10) for restoring the emission of the pulses being activated by the CPU after correct repositioning of the electrode.

3. Electrostimulator according to claim 1 or 2, **characterized in that** it further comprises a circuit (7,8,3) that automatically monitors the temperature on the surface in contact with the electrodes (1) and limits or blocks the flow of current, in case of an increase in skin temperature which is predetermined so as to prevent undesired reddening or burning of the skin itself due to excessive heating of the body part concerned

## Patentansprüche

1. Elektrischer Muskelstimulator, ausgebildet auf solche Weise, dass Schmerzen und Rötungen der Haut verhindert werden, enthaltend einen Impulserzeuger (2), Überwachungs- und Speisungskreise, Elektroden (1), Regelelemente (11) und einen Kreis (4, 5, 3) zum Steuern von Überspannung an den Elektroden, welcher, wenn die vorgegebene Grenze überschritten wird, die Stromabgabe innerhalb einer solchen Zeit absperrt, dass die Person nicht in die Lage kommt, Schmerz zu empfinden, sowie Mittel (10) zur Wiederherstellung der Impulserzeugung, nachdem die Elektrode wieder korrekt positioniert worden ist, **dadurch gekennzeichnet, dass** die genannten Mittel (10) zur Wiederherstellung der Impulserzeugung durch Erhöhung der Amplitude der Impulse von Null auf einen eingestellten Wert wirken, und zwar auf solche Weise, dass schmerzhafte Schocks vermieden werden, ohne dabei die Notwendigkeit zu haben, die Spannung durch die Regelelemente (11) von Hand auf Null stellen zu müssen, welche somit auf dem eingestellten optimalen Wert positioniert bleibt.

2. Elektrischer Muskelstimulator nach Patentanspruch 1, **dadurch gekennzeichnet, dass** er eine CPU (3) enthält, wobei die genannten Mittel (10) zur Wiederherstellung der Impulserzeugung nach der korrekten erneuten Positionierung der Elektrode durch die CPU aktiviert wird.

3. Elektrischer Muskelstimulator nach Patentanspruch 1 oder 2, **dadurch gekennzeichnet, dass** er weiter einen Kreis (7, 8, 3) enthält, der automatisch die Temperatur an der Oberfläche im Kontakt mit den Elektroden (1) steuert und den Stromfluss begrenzt oder absperrt, falls eine Erhöhung der Hauttemperatur auftreten sollte, und welcher dazu vorgesehen ist, unerwünschte Rötungen oder Verbrennungen der Haut selbst zu verhindern, zurückzuführen auf ein übermässiges Erhitzen des betreffenden Körperteils.

## Revendications

1. Electrostimulateur musculaire empêchant les sensations de douleur et les rougissures de la peau, comprenant un générateur d'impulsion (2), des circuits de monitorage et d'entrée, des électrodes (1), des composants de réglage (11) et un circuit (4, 5, 3) pour le monitorage des surtensions sur les électrodes qui, si l'on excède la limite prédéterminée, bloque la sortie de courant en un temps de durée telle que la personne ne soit pas capable de percevoir aucune douleur, **caractérisé en ce que** des moyens (10) pour restaurer l'émission d'impulsions après que l'électrode ait été correctement repositionnée, lesdits moyens (10) pour la restauration de l'émission d'impulsions agissant en augmentant l'amplitude des impulsions de zéro à une valeur déterminée de manière à prévenir les chocs douloureux sans la nécessité de régler le voltage sur zéro manuellement au moyen des composants de réglage (11) qui restent ainsi positionnés sur la valeur optimale présélectionnée.

2. Electrostimulateur selon la revendication 1, **caractérisé en ce qu'**il comprend une CPU (3), lesdits moyens (10) pour restaurer l'émission des impulsions étant activés par la CPU après que l'électrode ait été correctement repositionnée.

3. Electrostimulateur selon la revendication 1 ou 2, **caractérisé en ce qu'**il comprend en outre un circuit (7, 8, 3) qui contrôle automatiquement la température sur la surface en contact avec les électrodes (1) et limite ou bloque le flux de courant, dans le cas d'une augmentation de la température de la peau prédéterminée de manière à prévenir les brûlures ou rougissures de la peau dues à un réchauffement excessif de la partie du corps concernée.
